# EUROPEAN PATENT APPLICATION

(11) **EP 3 973 919 A1**
(43) Date of publication of application: **30.03.2022**
(21) Application number: 21190929.6
(22) Date of filing: 12.08.2021
(51) Int. Cl.: A61C 17/22, A61C 17/34, A46B 15/00, A61B 5/00, A61B 5/145, A61B 5/02

(54) **INTELLIGENT TOOTH HEALTH-CARE SYSTEM AND INTELLIGENT TOOTH CLEANING DEVICE THEREOF**

(30) Priority: 28.09.2020 TW 109133662
(71) Applicant: National Taiwan University of Science and Technology, Taipei City 10607 (TW)
(72) Inventor: JENG, Jeng-Ywan, 10607 Taiwan (TW); WU, Guan-Syun, 10607 Taiwan (TW); CHEN, I-An, 10607 Taiwan (TW); HAMZA, Azam, 10607 Taiwan (TW)
(74) Representative: Renaudo, Adrien Hanouar

(57) **Abstract**

An intelligent tooth health-care system and an intelligent tooth cleaning device thereof. The intelligent tooth cleaning device includes a plurality of sensors capable of detecting conditions of mouth and teeth of a user. The user may obtain data relating to his/her health when he/she cleans the teeth. The sensors also detect the movement of the intelligent tooth cleaning device . The data of movement are transmitted to a mobile device and input to a computer tooth-cleaning game executed in the mobile device, whereby the user is guided by the computer tooth-cleaning game to clean his/her teeth. The data obtained by the sensors can also be transmitted to a server in the cloud for health analysis.

## Description

### FIELD OF THE INVENTION

The present invention relates to the technical field of tooth health care, and in particular, to an intelligent tooth health-care system and an intelligent tooth cleaning device thereof.

### BACKGROUND OF THE INVENTION

The cleaning of teeth affects personal health greatly, and the most basic habit of tooth cleaning is the tooth brushing activity every morning and evening. In addition to an ordinary toothbrush, an electric toothbrush can also be used to brush teeth. However, in the process of brushing teeth, users cannot understand the conditions of their oral cavities and teeth, and thus do not carefully clean the required parts when brushing teeth. This often leads to dental and oral diseases, such as dental caries and periodontal diseases.

### SUMMARY OF THE INVENTION

In view of the above, an objective of the present invention is to provide an intelligent tooth health-care system and an intelligent tooth cleaning device thereof. The intelligent tooth cleaning device includes a variety of sensing components for detecting the status of the oral cavity and teeth, whereby data related to health conditions of a user are obtained while brushing teeth. The sensing components can also detect the status of movement of the tooth cleaning device itself to guide the user to clean teeth cooperating with a computer game according to the status of the oral cavity and teeth.

An exemplary embodiment of the intelligent tooth health-care system of the present invention includes a holding member, a tooth cleaning member, a first processor, a driver, a transmission member, a first wireless transceiver module, a displacement detection module, a saliva detection module and a camera module. The holding member has a first end and a second end. The tooth cleaning member has a brush head, a connecting part and a plurality of bristles, wherein the connecting part is connected to the brush head and detachably connected to the first end, and the bristles are arranged on the brush head. The first processor is disposed in the holding member. The driver is disposed in the holding member and electrically connected to the first processor. The transmission member extends outward from the first end of the holding member and connected the driver and the tooth cleaning member, wherein the torsion force of the driver is transmitted to the tooth cleaning member via the transmission member, so that the tooth cleaning member rotates or vibrates. The first wireless transceiver module is disposed in the holding member and electrically connected to the first processor. The displacement detection module is disposed in the holding member, electrically connected to the first processor, and configured to detect the position of the holding member and generate a displacement signal. The saliva detection module is disposed on the surface of the brush head of the tooth cleaning member, electrically connected to the first processor, and configured to detect the saliva of a toothbrush user and generate a physiological characteristic signal. The camera module is electrically connected to the first processor and configured to capture an image of the oral cavity of the toothbrush user from the brush head and generate an image signal. The displacement signal and the physiological characteristic signal are transmitted to the first processor via the first wireless transceiver module.

In another embodiment, the saliva detection module comprises a blood detector, an acid-base detector and a blood glucose detector, and the physiological characteristic signal comprises a blood concentration signal, a pH signal and a blood glucose concentration signal.

In another embodiment, the saliva detection module is detachably disposed on the surface of the brush head of the tooth cleaning member.

In another embodiment, the transmission member extends from the first end in a direction away from a shell, the connecting part is sleeved on the transmission member and the transmission member extends to the inside of the brush head, the camera module is disposed on the transmission member, and the brush head has a first transparent window corresponding to the camera module, and light outside the brush head enters the camera module through the first transparent window.

In another embodiment, the intelligent tooth health-care system of the present invention further includes a light-emitting module disposed on the extension part and electrically connected to the first processor, and the brush head further has a second transparent window corresponding to the light-emitting module, and light emitted from the light-emitting module exits the brush head through the second transparent window.

In another embodiment, the intelligent tooth health-care system of the present invention further includes a power module disposed in the shell of the holding member and electrically connected to the first processor.

In another embodiment, the holding member is detachably placed on the bearing base, the bearing base has a charging module, and the charging module is electrically connected to the power module and charges the power module when the holding member is placed on the bearing base.

Another objective of the present invention is to provide an intelligent tooth health-care system, including the intelligent tooth cleaning device and a mobile device. The mobile device includes a second processor, a display module, a storage module, and a second wireless transceiver module. The display module, the storage module, and the second wireless transceiver module are electrically connected to the second processor, the displacement signal and the physiological characteristic signal are transmitted from the first wireless transceiver module to the second wireless transceiver module, the displacement signal is transmitted to the second processor, and the second processor loads a computer game from the storage module and executes the computer game, and the displacement signal is input to the computer game.

In another embodiment, the intelligent tooth health-care system of the present invention further includes a cloud server, wherein the mobile device further comprises a third wireless transceiver module, and the physiological characteristic signal is transmitted through the third wireless transceiver module to the cloud server for health status analysis.

In another embodiment, the mobile device obtains tooth structure data of the toothbrush user according to the displacement signal and the image signal, and the mobile device generates a control signal according to the tooth structure data, the control signal is transmitted to the intelligent tooth cleaning device, and the intelligent tooth cleaning device performs tooth cleaning according to the control signal.

**The intelligent tooth cleaning device of the present invention can simultaneously detect the displacement of the intelligent tooth cleaning device and the status of saliva and capture images of the oral cavity when performing tooth cleaning, and combine the detected displacement signal, physiological characteristic signal and image signal into the tooth cleaning game performed on the mobile device to guide the user to clean the teeth. The mobile device also transmits the displacement signal, physiological characteristic signal and image signal to the cloud server for analysis, provides the analysis result to the dentist as a reference for diagnosis, and provides the user with a full range of tooth cleaning guidance, and can monitor own health status of the user in real time.**

### BRIEF DESCRIPTION OF THE DRAWINGS

FIG. 1 is a front view of an embodiment of an intelligent tooth cleaning device of the present invention.
FIG. 2 is an exploded view of the intelligent tooth cleaning device of FIG. 1.
FIG. 3 is a side view of the intelligent tooth cleaning device of FIG. 2.
FIG. 4 is a block diagram of an embodiment of an intelligent tooth health-care system of the present invention.
FIG. 5 is a schematic view of the intelligent tooth health-care system of the present invention.

### Major Components Symbol Description

- 10: Holding member
- 11: First end
- 12: Second end
- 13: Circuit board
- 20: Tooth cleaning member
- 21: Brush head
- 22: Connecting part
- 23: Bristle
- 30: Bearing base
- 40: First processor
- 50: Driver
- 60: Transmission member
- 70: Displacement detection module
- 80: Saliva detection module
- 90: Camera module
- 100: Intelligent tooth cleaning device
- 110: First light-emitting module
- 120: Power module
- 200: Mobile device
- 300: Cloud server

### DETAILED DESCRIPTION OF THE PREFERRED EMBODIMENTS

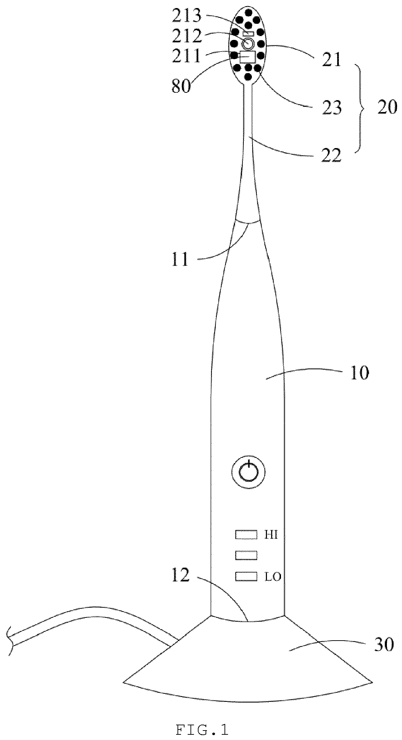

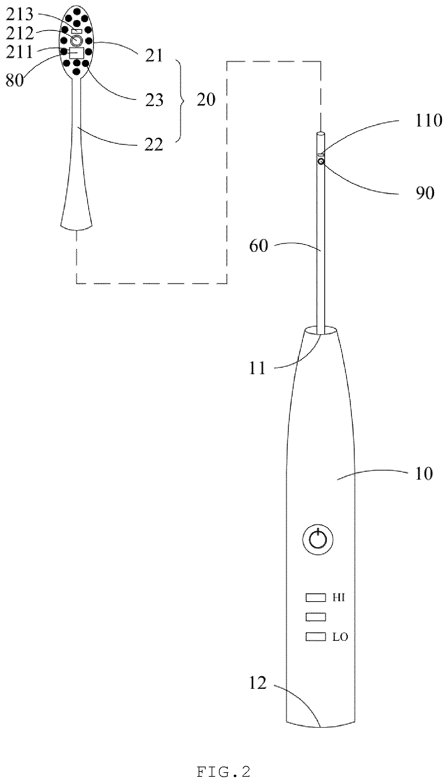

Referring to FIGS. 1, 2 and 3, an embodiment of an intelligent tooth cleaning device of the present invention is shown. As shown in FIG. 2, the intelligent tooth cleaning device 100 of the present invention includes a holding member 10, a tooth cleaning member 20 and a bearing base 30.

As shown in FIG. 1, the holding member 10 has a cylindrical shell, for example, a cylindrical shape or an elliptical shape for the user's hand to hold. The holding member 10 has a first end 11 and a second end 12, and the tooth cleaning member 20 is connected to the first end 11. The second end 12 of the holding member 10 can be placed on the bearing base 30. The tooth cleaning member 20 has a brush head 21, a connecting part 22 and a plurality of bristles 23. The connecting part 22 is connected to the brush head 21 and detachably connected to the first end 11, and the bristles 23 are arranged on the brush head 21. The shape of the connecting part 22 can be matched with the holding member 10, and the connecting part 22 can be made of elastic and flexible materials, so that the tooth cleaning member 20 can easily generate rotation or vibration as described later for cleaning teeth.

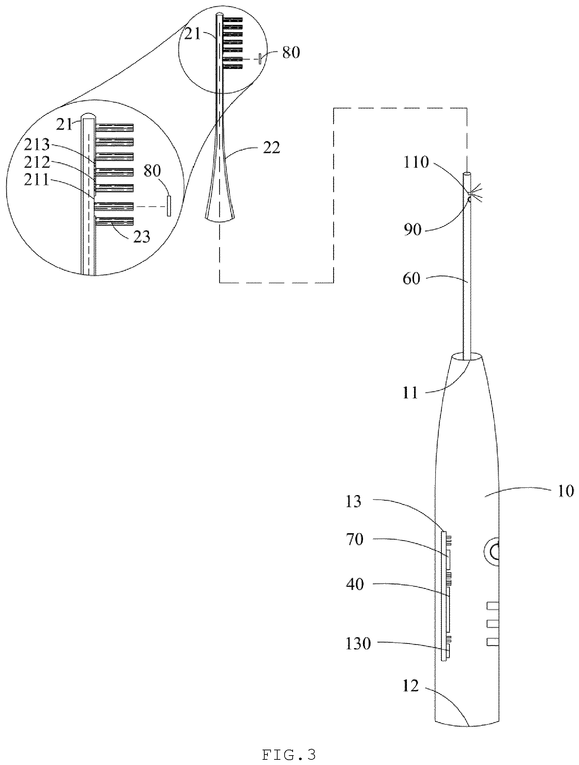

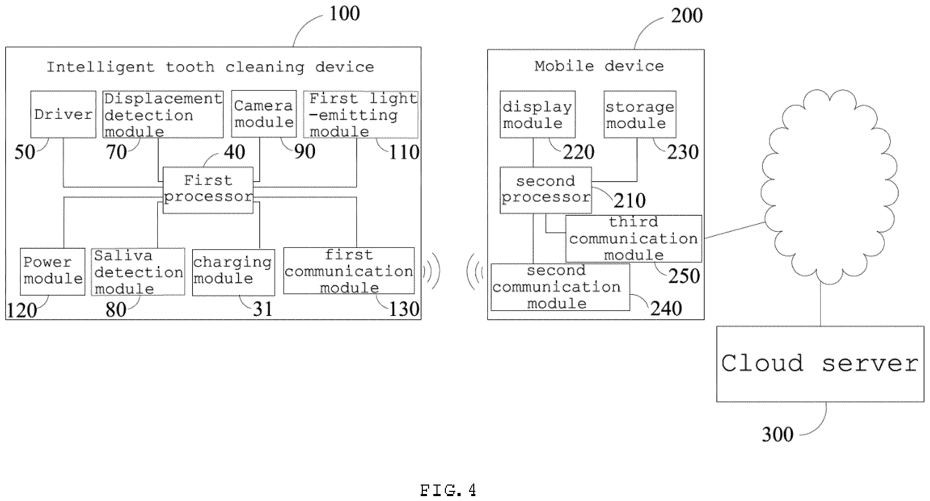

As shown in FIGS. 2 and 3, and referring to FIG. 4, the intelligent tooth cleaning device 100 of the present invention includes a first processor 40, a driver 50, a transmission member 60, and a displacement detection module 70, a saliva detection module 80, a camera module 90, a first light-emitting module 110, and a power module 120. A circuit board 13 is disposed in the shell of the holding member 10. The first processor 40 and the displacement detection module 70 are disposed on the circuit board 13. As shown in FIG. 2, a power switch and a speed control button are provided on a surface of the shell. The power switch and the speed control button are electrically connected to the first processor 40. A control signal is generated by pressing the power switch and the speed control button, and the control signal is transmitted to the first processor 40. The first processor 40 can control the ON/OFF of a power supply and control the speed of rotation or vibration of the tooth cleaning member 20.

The driver 50 is disposed in the shell of the holding member 10, and the transmission member 60 is disposed in the shell and connected to the driver 50. As shown in FIGS. 2 and 3, the transmission member 60 extends from the first end 11 of the holding member 10 to the outside of the shell. The tooth cleaning member 20 is sleeved on the transmission member 60, and the transmission member 60 extends to the interior of the tooth cleaning member 20. In this embodiment, the driver 50 is an electric motor, and the transmission member 60 is an extension rod. The driver 50 converts the torsion force into the rotation of the transmission member 60 or the vibration orthogonal to an extension direction of the transmission member 60 through the transmission module, whereby the tooth cleaning member 20 generates rotation or vibration to clean the teeth.

As shown in FIG. 4, the power module 120 is disposed in the shell of the holding member 10 and electrically connected to the first processor 40. The power of the power module 120 is provided to the driver 50, the displacement detection module 70, the saliva detection module 80, the camera module 90 and the first light-emitting module 110 via the first processor 40. The power module 120 is a rechargeable battery. The power module 120 is disposed at the second end 12 close to the holding member 10. As described above, the second end 12 can be placed on the bearing base 30. A charging module 31 is provided in the bearing base 30 and connected to the mains. Metal conductive sheets or coils are respectively provided at the second end 12 and the bearing base 30 and respectively connected to the power module 120 and the charging module. The charging module 31 of the bearing base 30 can charge the power module 120 through the contact of the metal conductive sheets or the electromagnetic interaction of the coils when the second end 12 is placed on the bearing base 30.

The displacement detection module 70 is disposed on a circuit board within the holding member 10. The displacement detection module 70 includes an accelerometer and a gyroscope. When the user holds the holding member 10 for brushing teeth, the accelerometer and gyroscope can detect the direction of movement and rotation of the holding member 10 and generate a displacement signal, and the displacement signal is transmitted to the first processor 40.

As shown in FIGS. 2, 3, and 4, the saliva detection module 80 includes a blood detector, an acid-base detector, and a blood glucose detector, and contacts the saliva to detect the blood content, PH, and blood sugar levels in the saliva so as to generate a plurality of physiological characteristic signals. Therefore, the saliva detection module 80 is disposed on the tooth cleaning member 20, and saliva can be detected while the user extends the tooth cleaning member 20 into the oral cavity for cleaning the teeth. As shown in FIG. 3, in this embodiment, a recess 211 is formed on a surface of the brush head 21 where the bristles 23 are disposed, the recess 211 can be surrounded by the bristles 23, and the saliva detection module 80 is disposed in the recess 211 and can be detached from the brush head 21. When the user replaces the tooth cleaning member 20 since a portion of the bristles 23 fall off or bend after being used for a period of time, the saliva detection module 80 can be detached from the old brush head 21 and mounted to a new tooth cleaning member 20 which is to be installed on the holding member 10, so that the saliva detection module 80 can be reused. Wires can be arranged in the tooth cleaning member 20 to transmit the physiological characteristic signal generated by the saliva detection module 80 to the first processor 40, or a wireless transmission component may be disposed in the saliva detection module 80 to transmit the physiological characteristic signal to the first processor 40.

The camera module 90 is used to capture images of the oral cavity and teeth. The first light-emitting module 110 generates light for illuminating the oral cavity. The camera module 90 cooperates with the first light-emitting module 110 to obtain the image of the oral cavity of the user in the tooth cleaning process. Therefore, the camera module 90 and the first light-emitting module 110 are preferably disposed at the positions corresponding to the tooth cleaning member 20. As described above, the transmission member 60 extends to the interior of the tooth cleaning member 20 through the first end 11 of the holding member 10 and extends to the interior of the brush head 21 through the connecting part 22. Therefore, in this embodiment, the camera module 90 and the first light-emitting module 110 are disposed at the positions close to the rear end of the transmission member 60 and corresponding to the brush head 21. A first transparent window 212 and a second transparent window 213 are provided on a surface of the brush head 21 where the bristles 23 are arranged. When the transmission member 60 extends to the interior of the tooth cleaning member 20, the camera module 90 corresponds to the first transparent window 212, the first light-emitting module 110 corresponds to the second transparent window 213, the light emitted from the first light-emitting module 110 is irradiated to the exterior of the brush head 21 through the second transparent window 213, and the light outside the brush head 21 enters the camera module 90 through the first transparent window 212 to generates images. Wires can be arranged inside the transmission member 60 to electrically connect the camera module 90 and the first light-emitting module 110 to the first processor 40. The power of the power module 120 can be transmitted to the camera module 90 and the first light-emitting module 110 through the first processor 40. The image signal generated by the camera module 90 can be transmitted to the first processor 40 via the wires. The first light-emitting module 110 may include a plurality of light-emitting components capable of emitting visible light while the camera module 90 receives the same and generates visible light images. The light-emitting components can also emit light of special wavelengths. After light is irradiated on the teeth, the dental plaque on the teeth may produce a special color, thus obtaining an image of the dental plaque distribution in the oral cavity.

In addition, a pressure sensor can also be disposed on the brush head 21 of the tooth cleaning member 20 to detect the pressure on the gingiva and teeth of the user when cleaning the teeth.

As shown in FIGS . 4 and 5, the intelligent tooth cleaning device 100 of the present invention further includes a first communication module 130, which is disposed on the circuit board in the holding member 10 and electrically connected to the first processor 40 After the displacement signal is generated by the displacement detection module 70, the physiological characteristic signal generated by the saliva detection module 80, and the image signal generated by the camera module 90 are transmitted to the first processor 40, the first processor 40 transmits the displacement signal, the physiological characteristic signal and the image signal to the mobile device 200 via the first communication module 130.

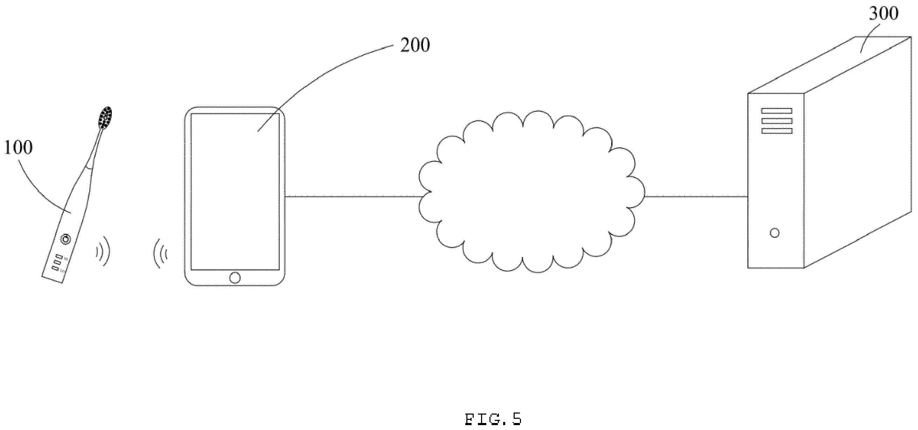

As shown in FIGS. 4 and 5, the intelligent tooth health-care system of the present invention includes the intelligent tooth cleaning device 100 of the present invention, a mobile device 200, and a cloud server 300. The mobile device 200 includes a second processor 210, a display module 220, a storage module 230, a second communication module 240, and a third communication module 250. The storage module 230 stores a program code of a computer game. The computer game includes for example, tooth cleaning game, tooth brushing game, tooth caring software or sanitation education program, but the computer game is not limited to them. The tooth cleaning game is described as an example for the embodiment. The second processor 210 loads the program code from the storage module 230 for execution and displays a game picture on the display module 220 when the user executes the tooth cleaning game on the mobile device 200. For example, a different monster is set at the position of each tooth. The user can set a game character. When each tooth is cleaned, the game picture shows the game character fighting each monster. When the user completes the cleaning action of each tooth, the monster is destroyed and a score is obtained for clearance. For teeth that are easier to clean, such as incisors or canines, the monsters that are easier to defeat and lower clearance scores can be set. However, for the teeth that are less easy to clean, such as molars, more powerful monsters and higher clearance scores can be set.

As described above, the displacement signal, the physiological characteristic signal and the image signal generated by the intelligent tooth cleaning device 100 are transmitted to the mobile device 200, and the displacement signal, the physiological characteristic signal and the image signal can be stored in the storage module 230. After obtaining the displacement signal, the mobile device 200 can calculate the current position of the intelligent tooth cleaning device 100, whereby the game level for the game character can be correspondingly displayed on the tooth cleaning game, and the moving trajectory of the intelligent tooth cleaning device 100 moved by the user can be obtained. Moreover, a plurality of physiological characteristic signals can also be used in tooth cleaning game. For example, when a higher blood concentration is detected at a certain position of the oral cavity, it is indicated that the oral cavity may be bleeding there in that position. In order to prevent the user from brushing his teeth to touch the bleeding position to worsen the bleeding, when the user moves the intelligent tooth cleaning device 100 to the tooth at that position, the tooth cleaning game can provide clearance treasures in real time, whereby the game character can immediately defeat the monster as soon as he arrives at that position for clearance. Similarly, the image signal can also be used in the tooth cleaning game. As described above, dental plaque can be detected by the light of a special wavelength. Therefore, the user is expected to do more cleaning on the teeth where the dental plaque is formed than the normal tooth cleaning, and thus, the tooth cleaning game can generate monsters with high scores in real time at the position corresponding to the teeth with dental plaque in the game interface image, allowing the user to perform more detailed and complete cleaning actions on the teeth. After the pressure signal generated by the pressure sensor is transmitted to the mobile device 200, it can also be used in the tooth cleaning game. For example, when the force of cleaning teeth is too large, it can directly cause reduction of the blood volume of the game character.

In addition, the mobile device 200 can transmit the displacement signal, the physiological characteristic signal and the image signal to a cloud server 300 via the third communication module 250. The cloud server 300 can construct a three-dimensional model of the oral cavity based on the image signal and mark the location of oral diseases on the three-dimensional model, such as dental caries, gingival or oral bleeding, and gingival cysts. The cloud server 300 can be connected to the user' s dentist at the same time, whereby the dentist can perform diagnosis in real time. The cloud server 300 can analyze information about the user's tooth cleaning habits based on the daily displacement signals transmitted by the mobile device 200. The information about the tooth cleaning habits is transmitted to the user's dentist at the same time, and the dentist can correct the user's tooth cleaning habits according to the three-dimensional image of the oral cavity. After the physiological characteristic signal is transmitted to the cloud server 300, the saliva pH value and blood glucose value can be used as a reference for the user's daily health data.

In another embodiment, since the user has to hold the holding member 10 with a hand for cleaning teeth, a body temperature sensor, a heart rate sensor, and a blood pressure sensor, etc. can be provided on the holding member 10. When the user holds the holding member 10, the user's body temperature data, heart rate data, blood pressure and other physiological characteristic signals can be simultaneously measured and transmitted to the cloud server 300 via the mobile device 200, synchronously.

In another embodiment, an ultrasonic component can be disposed on the tooth cleaning member 20. When the user places the tooth cleaning member 20 in the oral cavity, the ultrasonic component can produce vibration to loosen dental calculus, whereby the dental calculus can be removed while cleaning teeth.

In another embodiment, a dentifrice storage space can be formed on the tooth cleaning member 20. In conjunction with the oral images captured by the camera module 90, when the tooth cleaning member 20 is moved to a position that requires special cleaning, such as teeth with dental plaque or teeth with a particularly yellow color, the first processor 40 controls a micro pump disposed on the holding member 10 to pressurize the dentifrice from the tooth cleaning member 20 to achieve the effect of eliminating dental plaque or enhancing cleaning.

In addition to a slot for accommodating the holding member 10, the bearing base 30 can be provided with a slot for accommodating the mobile device 200. When the user picks up the holding member 10, the mobile device 200 can be placed on the bearing base 30, the slot for accommodating the mobile device 200 on the supporting base 30 can also be connected to a charging circuit to charge the mobile device 200, and the mobile device 200 communicates with the intelligent tooth cleaning device 100 at the same time.

In another embodiment, the intelligent tooth cleaning device 100 further includes a second light-emitting module, which is disposed on the transmission member 60 and located on the opposite side of the first light-emitting module 110. When the transmission member 60 is disposed in the tooth cleaning member 20, the second light-emitting module corresponds to the back of the brush head 21, and the back is relative to the surface of the brush head 21 where the bristles 23 are arranged. A third transparent window can be provided on the back of the brush head 21, and light emitted from the second light-emitting module can be emitted through the third transparent window. Since the second light-emitting module is disposed on the back of the brush head 21, when cleaning teeth, the second light-emitting module faces the mucosa of the oral cavity. If the oral cavity of the user has an affected part such as oral inflammation, ulceration or oral cancer, the second light-emitting module can emit therapeutic light to irradiate the affected area for treatment while cleaning the teeth.

In another embodiment, the bearing base 30 of the intelligent tooth cleaning device 100 includes a third processor, a display module, and a storage module. The tooth cleaning game is stored in the storage module of the bearing base 30 and loaded and executed by the third processor to display the game picture on the display module . The displacement signal, the physiological characteristic signal and the image signal are transmitted to the bearing base 30 through the first communication module and matched with the tooth cleaning game. In this embodiment, the intelligent tooth cleaning device 100 can guide the user to clean the teeth with the tooth cleaning game, without connecting to the mobile device 200.

In addition, in another embodiment, the intelligent tooth cleaning device 100 of the present invention can clean teeth in different locations in a manner according to the status of each tooth. As described above, since the intelligent tooth cleaning device 100 of the present invention has the displacement detection module 70 and the camera module 90, when the user uses the intelligent tooth cleaning device 100 of the present invention for the first time, as the user moves the intelligent tooth cleaning device 100 of the present invention in the oral cavity, the displacement detection module 70 and the camera module 90 can respectively detect the position and image of each tooth, which can be transmitted to the mobile device 200 and are used, in cooperation with an application program, to construct various data of the tooth structure in the oral cavity, such as the position coordinates of each tooth, the width and length of the tooth, the width of the slit between the teeth, and the area of the occlusal surface.

After the construction of various data of the tooth structure in the oral cavity is completed, when the user uses the intelligent tooth cleaning device 100 of the present invention for brushing teeth again, the mobile device 200 is informed which tooth the intelligent tooth cleaning device 100 moves to according to the displacement signal transmitted by the intelligent tooth cleaning device 100, and accordingly, performs tooth cleaning in different tooth cleaning modes. For example, the rotation frequency of the driver 50 is changed to allow the brush head 21 to vibrate at different frequencies, or the intelligent tooth cleaning device 100 moves to the slit between the teeth, and the driver 50 can drive the brush head 21 to vibrate at a larger amplitude, so as to clean the deep slit between the teeth. In addition, when the intelligent tooth cleaning device 100 moves to the junction between the tooth and the gingiva, the driver 50 can drive the brush head 21 to clean the junction between the tooth and the gingiva with greater pressure to avoid the accumulation of the dental plaque and form dental calculus, so as to prevent periodontal diseases. In addition, when the user wants to whiten the teeth, the dentifrice can be discharged, and the driver 50 can drive the brush head 21 to vibrate at a high frequency to intensively clean the tooth surface to achieve the effect of cleaning and whitening.

When the intelligent tooth cleaning device 100 performs tooth cleaning in various modes, the camera module 90 continues to capture images of the oral cavity and teeth. The mobile device 200 can determine the status of each tooth to be cleaned according to the image signal transmitted by the camera module 90 and use it as a basis for adjust the operation of the driver 50 for each tooth in the following tooth cleaning performance on the basis of the status of each tooth to change the action of the brush head 21. Therefore, the intelligent tooth cleaning device 100 of the present invention can learn according to the status of the user's oral cavity to obtain the best tooth cleaning mode for each tooth and perform the tooth cleaning according to the best mode for each tooth during tooth cleaning, to achieve the purpose of intelligent tooth cleaning.

The intelligent tooth cleaning device of the present invention can simultaneously detect the displacement of the intelligent tooth cleaning device and the status of saliva and capture images of the oral cavity when performing tooth cleaning, and combine the detected displacement signal, physiological characteristic signal and image signal into the tooth cleaning game performed on the mobile device to guide the user to clean the teeth. The mobile device also transmits the displacement signal, physiological characteristic signal and image signal to the cloud server for analysis, provides the analysis result to the dentist as a reference for diagnosis, and provides the user with a full range of tooth cleaning guidance, and can monitor own health status of the user in real time.

However, the above are only preferred embodiments of the present invention and should not be construed as limiting the scope of the present invention. That is, simple equivalent variations and modifications made according to the claims and description of the present invention still fall within the scope of protection of the present invention.

## Claims

1. An intelligent tooth cleaning device, comprising:
a holding member having a first end and a second end;
a tooth cleaning member having a brush head, a connecting part and a plurality of bristles, wherein the connecting part is connected to the brush head and detachably connected to the first end, and the bristles are arranged on the brush head;
a first processor disposed in the holding member;
a driver disposed in the holding member and electrically connected to the first processor;
a transmission member extending outward from the first end of the holding member and connected the driver and the tooth cleaning member, wherein the torsion force of the driver is transmitted to the tooth cleaning member via the transmission member to rotate or vibrate the tooth cleaning member;
a first wireless transceiver module disposed in the holding member and electrically connected to the first processor;
a displacement detection module disposed in the holding member, electrically connected to the first processor, and configured to detect the position of the holding member and generate a displacement signal;
a saliva detection module disposed on the surface of the brush head of the tooth cleaning member, electrically connected to the first processor, and configured to detect the saliva of a toothbrush user and generate a physiological characteristic signal; and
a camera module electrically connected to the first processor and configured to capture an image of the oral cavity of the toothbrush user from the brush head and generate an image signal;
wherein the displacement signal and the physiological characteristic signal are transmitted to the first processor via the first wireless transceiver module.

2. The intelligent tooth cleaning device according to claim 1, wherein the saliva detection module comprises a blood detector, an acid-base detector and a blood glucose detector, and the physiological characteristic signal comprises a blood concentration signal, a pH signal and a blood glucose concentration signal.

3. The intelligent tooth cleaning device according to claim 1, wherein the saliva detection module is detachably disposed on a surface of the brush head of the tooth cleaning member.

4. The intelligent tooth cleaning device according to claim 1, wherein the transmission member extends from the first end in a direction away from the holding member, the connecting part is sleeved on the transmission member and the transmission member extends to an interior of the brush head, the camera module is disposed on the transmission member, and the brush head has a first transparent window corresponding to the camera module, and light outside the brush head enters the camera module through the first transparent window.

5. The intelligent tooth cleaning device according to claim 4, further comprising a light-emitting module disposed on the transmission member and electrically connected to the first processor, and the brush head further has a second transparent window corresponding to the light-emitting module, and light emitted from the light-emitting module exits the brush head through the second transparent window.

6. The intelligent tooth cleaning device according to claim 1, further comprising a power module disposed in a shell of the holding member and electrically connected to the first processor.

7. The intelligent tooth cleaning device according to claim 6, further comprising a bearing base, wherein the holding member is detachably placed on the bearing base, the bearing base has a charging module, and the charging module is electrically connected to the power module and charges the power module when the holding member is placed on the bearing base.

8. An intelligent tooth health-care system, comprising:
the intelligent tooth cleaning device according to any one of claims 1; and
a mobile device comprising a second processor, a display module, a storage module, and a second wireless transceiver module, wherein the display module, the storage module, and the second wireless transceiver module are electrically connected to the second processor; the displacement signal and the physiological characteristic signal are transmitted from the first wireless transceiver module to the second wireless transceiver module, the displacement signal is transmitted to the second processor, and the second processor loads a computer game from the storage module and executes the computer game, and the displacement signal is input to the computer game.

9. The intelligent tooth health-care system according to claim 8, further comprising a cloud server, wherein the mobile device further comprises a third wireless transceiver module, and the physiological characteristic signal is transmitted through the third wireless transceiver module to the cloud server for health status analysis.

10. The intelligent tooth health-care system according to claim 8, wherein the mobile device obtains tooth structure data of the toothbrush user according to the displacement signal and the image signal, and the mobile device generates a control signal according to the tooth structure data, the control signal is transmitted to the intelligent tooth cleaning device, and the intelligent tooth cleaning device performs tooth cleaning according to the control signal.
